# EUROPÄISCHE PATENTANMELDUNG

(11) **EP 4 717 203 A1**
(43) Veröffentlichungstag der Anmeldung: **01.04.2026**
(21) Anmeldenummer: 25202061.5
(22) Anmeldetag: 15.09.2025
(51) Int. Cl.: A61B 17/80

(54) **OSTEOSYNTHESEPLATTE SOWIE VERFAHREN ZUR HERSTELLUNG EINER OSTEOSYNTHESEPLATTE**

(30) Priorität: 25.09.2024 DE 102024127782
(71) Anmelder: Karl Leibinger Asset Management GmbH & Co. KG, 78570 Mühlheim an der Donau (DE)
(72) Erfinder: Pabst, Martin, 78479 Reichenau (DE); Meer, Martin, 72189 Vöhringen (DE)
(74) Vertreter: Schwamberger, Martin

(57) **Zusammenfassung**

Osteosyntheseplatte (P) zur Fixierung von Knochenfragmenten, wobei die Osteosyntheseplatte (P) einen länglichen Grundkörper (G) mit einer Oberseite (G1) und mit einer gegenüberliegenden Unterseite (G2) aufweist, wobei die Unterseite (G2) zur Auflage der Osteosyntheseplatte (P) auf den Knochenfragmenten vorgesehen ist, wobei die Osteosyntheseplatte (P) mehrere Öffnungen (A) zur Aufnahme von je einer Schraube (SK) aufweist, wobei in zumindest einer der Öffnungen (A) ein einteilig und ringförmig ausgebildeter Einsatz (R) angeordnet ist, wobei der Einsatz (R) einen ersten und einen zweiten Abschnitt (R1, R2) aufweist, wobei der erste Abschnitt (R1) der Oberseite (G1) zugewandt ist und in axialer Richtung unmittelbar auf den zweiten Abschnitt (R2) anschließend angeordnet ist, wobei der erste Abschnitt (R1) eine nichtrotationssymmetrische Außenkontur (AD1) aufweist und der zweite Abschnitt (R2) eine rotationssymmetrische Außenkontur (AD2) aufweist, sowie Verfahren zur Herstellung einer solchen Osteosyntheseplatte (P).

## Beschreibung

Die Erfindung betrifft eine Osteosyntheseplatte zur Fixierung von Knochenfragmenten, sowie ein Verfahren zur Herstellung einer solchen Osteosyntheseplatten.

Osteosyntheseplatten werden in der Human- und Veterinärmedizin zur Fixierung von Knochenfragmenten verwendet, beispielsweise nach einer Fraktur, um das Zusammenwachsen der Knochenfragmente in einer anatomisch korrekten Lage zu fördern.

So lehrt die WO 2017/139903 A1 beispielsweise eine Vorrichtung zur Knochenfixation mit einer Knochenplatte, welches ein Plattenloch aufweist. Im Plattenloch ist ein hohlzylinder- oder hohlkegelförmiger Einsatz gelagert, welcher zur Aufnahme des Kopfes einer Knochenschraube vorgesehen ist. Der Einsatz ist dabei durch einen Formschluss verdrehsicher im Plattenloch angeordnet, beispielsweise mittels Vorsprünge in der Innenwand des Plattenlochs und entsprechende Einkerbungen im Einsatz.

Die EP 2 168 513 A1 zeigt eine Osteosynthesevorrichtung mit einer Platte, welche zumindest ein Durchgangsloch aufweist. Die Platte weist um das Durchgangsloch eine Einlage auf, welches aus einem weicheren Material als die Platte gebildet ist. Die Einlage und die Platte sind formschlüssig miteinander verbunden. Zur Verdrehsicherung der Einlage in Bezug auf die Platte wird vorgeschlagen, dass die Einlage nicht vollständig rotationssymmetrisch ist.

Lösungen mit derartigen Einsätzen, bzw. Einlagen in der Platte ermöglichen die Verwendung eines duktileren Materials im Bereich der Durchgangslöcher im Vergleich zum übrigen Körper der Platte. Durch Verwendung des duktileren Materials im Bereich der Durchgangslöcher ist kein vorgeformtes Innengewinde erforderlich, welches die Winkel-Variabilität der Schraubrichtung einschränken würde. Allerdings reduzieren die Einsätze, bzw. Einlagen den im Grundkörper zwischen den Durchgangslöchern zur Verfügung stehenden Querschnitt, sodass die Anzahl der Durchgangslöcher begrenzt wird.

Es ist daher eine erste Aufgabe der Erfindung, eine Osteosyntheseplatte bereitzustellen, welche sich durch einen möglichst kleinen Bauraumbedarf der Einsätze auszeichnet. Eine weitere Aufgabe der Erfindung ist es, ein prozesssicheres Verfahren zur Herstellung einer solchen Osteosyntheseplatte anzugeben.

Die erste Aufgabe wird gelöst durch die Merkmale des Patentanspruchs 1. Die weitere Aufgabe wird gelöst durch die Merkmale des Patentanspruchs 15. Vorteilhafte Ausgestaltungen ergeben sich aus den abhängigen Patentansprüchen, der Beschreibung sowie aus den Figuren.

Zur Lösung der ersten Aufgabe wird eine Osteosyntheseplatte zur Fixierung von Knochenfragmenten vorgeschlagen, welche einen länglichen Grundkörper mit einer Oberseite und mit einer der Oberseite gegenüberliegenden Unterseite aufweist. Die Unterseite ist zur Auflage der Osteosyntheseplatte auf den Knochenfragmenten vorgesehen. Die Osteosyntheseplatte weist mehrere Öffnungen zur Aufnahme von je einer Schraube auf, vorzugsweise zur Aufnahme eines Schraubenkopfs mit daran ausgeformtem Gewinde. In zumindest einer der Öffnungen ist ein einteilig und ringförmig ausgebildeter Einsatz angeordnet. Der Einsatz weist einen ersten Abschnitt und einen zweiten Abschnitt auf, welche in axialer Richtung unmittelbar anschließend zueinander angeordnet sind. Unter "axialer Richtung" ist hierbei die Richtung einer Mittelachse des Einsatzes gemeint. Der erste Abschnitt ist der Oberseite zugewandt, sodass der zweite Abschnitt der Unterseite zugewandt ist. Der erste Abschnitt weist eine nicht-rotationssymmetrische Außenkontur auf. Der zweite Abschnitt weist eine rotationssymmetrische Außenkontur auf.

Durch die Aufteilung des Einsatzes in einen ersten Abschnitt mit nichtrotationssymmetrischer Außenkontur und in einen zweiten Abschnitt mit rotationssymmetrischer Außenkontur erfolgt erfindungsgemäß eine Funktionsaufteilung - der erste Abschnitt dient zur Verdrehsicherung des Einsatzes relativ zum Grundkörper, während der zweite Abschnitt zur Axialsicherung des Einsatzes dient. Durch diese Funktionsaufteilung können die beteiligten Querschnitte für die jeweilige Aufgabe optimiert werden, sodass der Bauraumbedarf der Einsätze minimiert wird.

Vorzugsweise wird die nicht-rotationssymmetrische Außenkontur des ersten Abschnitts des Einsatzes durch zumindest sechs gleichmäßig oder ungleichmäßig am Umfang des Einsatzes verteilte Erhebungen gebildet. Der Begriff "Erhebungen" bezieht sich dabei nur auf die Form, und nicht auf den Herstellprozess. Durch die zumindest sechs Erhebungen wird eine besonders gleichmäßige Kraftverteilung bei der Drehmomentabstützung des Einsatzes relativ zum Grundkörper der Osteosyntheseplatte ermöglicht, sodass der erforderliche Querschnitt des ersten Abschnitts des Einsatzes gering gehalten werden kann. Eine gleichmäßige Verteilung der Erhebungen entlang dem Umfang bietet den Vorteil, dass das Einlegen des Einsatzes in den Grundkörper in verschiedenen Winkelausrichtungen erfolgen kann, sodass die Montage der Osteosyntheseplatte vereinfacht wird. Eine ungleichmäßige Verteilung der Erhebungen kann von Vorteil sein, wenn eine definierte Winkelausrichtung des Einsatzes beim Einlegen in die Osteosyntheseplatte gewünscht ist. Dies ist beispielsweise der Fall, wenn an der zur Oberseite gerichteten Oberfläche des Einsatzes eine Farbcodierung mit mehreren Farben vorgesehen ist. Um die derartig farbig markierten Einsätze gleichartig zueinander auszurichten, ist eine definierte Winkellage bei der Montage von Vorteil.

Gemäß einer bevorzugten Ausgestaltung weist die nicht-rotationssymmetrische Außenkontur des ersten Abschnitts des Einsatzes genau acht oder genau neun Erhebungen auf. Diese Zahl hat sich in Versuchen als ein guter Kompromiss zwischen einfacher und zuverlässiger Fertigung, kompakter Konstruktion und einfacher Montage ausgezeichnet.

Vorzugsweise bilden die Erhebungen einen größeren Anteil der nicht-rotationssymmetrischen Außenkontur als die sich in Umfangsrichtung zwischen den Erhebungen erstreckenden Täler. In anderen Worten sind die Täler schmäler als die Erhebungen. Dadurch wird der Querschnitt des ersten Abschnitts des Einsatzes besonders gut ausgenutzt.

Vorzugsweise weist jede der Erhebungen einen kreisbogenförmigen Abschnitt auf, dessen Zentrum koaxial zur Mittelachse des Einsatzes ist. Eine derartige Form der Erhebungen verbessert die Kräfteverteilung innerhalb der Erhebungen bei der Drehmomentabstützung.

Vorzugsweise ist ein Verhältnis zwischen dem kleinsten Durchmesser der zwischen den Erhebungen angeordneten Abschnitte und dem größten Durchmesser der Erhebungen größer als 0,8, bevorzugt größer als 0,85, besonders bevorzugt größer als 0,9. Durch ein solches Verhältnis kann der ringförmige Einsatz besonders dünnwandig gestaltet werden.

Gemäß einer bevorzugten Ausgestaltung ist die nicht-rotationssymmetrische Außenkontur wellenförmig ausgebildet. Vorzugsweise verlaufen die Übergange zwischen den Wellenbergen, i.e. die Erhebungen, und den Wellentälern in tangentialer Richtung. Eine derartige Form der Erhebungen verbessert die Kräfteverteilung innerhalb der Erhebungen bei der Drehmomentabstützung.

Vorzugsweise ist ein Übergang zwischen dem ersten und zweiten Abschnitt des Einsatzes durch eine Fläche gebildet ist, welche orthogonal zur Mittelachse des Einsatzes ausgerichtet ist. Diese Fläche kann zur Axialsicherung des Einsatzes dienen, beispielsweise indem die Fläche an einem radial nach innen gerichteten Vorsprung der Öffnung oder an einer Stufe der Öffnung anliegt.

Gemäß einer bevorzugten Ausgestaltung weist die Öffnung einen radial nach innen gerichteten Vorsprung auf, welcher eine zur Oberseite der Osteosyntheseplatte gerichtete erste Fläche und eine zur Unterseite der Osteosyntheseplatte gerichtete zweite Fläche aufweist. Der zweite Abschnitt des Einsatzes liegt in dieser Ausgestaltung zumindest bereichsweise sowohl auf der ersten Fläche als auch auf der zweiten Fläche auf. In anderen Worten umgreift der zweite Abschnitt den Vorsprung von beiden Seiten, sodass eine Axialsicherung des Einsatzes in beide Axialrichtungen erfolgt.

Vorzugsweise weist der Einsatz eine rotationssymmetrische Innenkontur mit nicht-konstantem Innendurchmesser auf. Unter einem "nicht-konstantem Innendurchmesser" wird hierbei verstanden, dass zumindest ein Abschnitt der Innenkontur einen nicht-konstanten Durchmesser aufweist. Die Innenkontur kann abschnittsweise einen konstanten Durchmesser aufweisen, allerdings nicht über die gesamte axiale Erstreckung der Innenkontur.

Vorzugsweise weist der Einsatz einen ersten Abschnitt mit konstantem Durchmesser auf. Dieser erste Abschnitt bildet den kleinsten Durchmesser der Innenkontur. Bei der Verschraubung der Osteosyntheseplatte mit den Knochenfragmenten formt sich das Gewinde der Knochenschraube, insbesondere das am Schraubenkopf geformte Gewinde, in diesen ersten Abschnitt ein.

Vorzugsweise weist die Innenkontur ausgehend vom ersten Abschnitt in Richtung der Oberseite der Osteosyntheseplatte einen zweiten Abschnitt auf. In Richtung der Unterseite der Osteosyntheseplatte weist die Innenkontur einen dritten Abschnitt auf. Der zweite und dritte Abschnitt bilden zumindest bereichsweise eine linear kegelig geformte Innenkontur mit einem ersten Kegelwinkelbereich. In anderen Worten weist die Innenkontur auf beiden Stirnseiten des ersten Abschnitts je eine Senkung auf. Dadurch wird der Materialfluss beim Einformen des Gewindes in den ersten Abschnitt verbessert. Der erste Kegelwinkelbereich kann beispielsweise zwischen 10 und 30 Grad betragen. Der Kegelwinkel des zweiten Abschnitts kann gleich oder unterschiedlich zum Kegelwinkel des dritten Abschnitts sein.

Vorzugsweise weist die Innenkontur ausgehend vom zweiten Abschnitt in Richtung der Oberseite der Osteosyntheseplatte einen weiteren Abschnitt auf, welcher zumindest bereichsweise eine linear kegelig geformte Kontur mit einem zweiten Kegelwinkelbereich bildet. Alternativ oder ergänzend dazu kann die Innenkontur ausgehend vom dritten Abschnitt in Richtung der Unterseite der Osteosyntheseplatte einen weiteren Abschnitt aufweisen, welcher zumindest bereichsweise eine linear kegelig geformte Kontur mit einem zweiten Kegelwinkelbereich bildet. Der zweite Kegelwinkelbereich ist dabei steiler als der erste Kegelwinkelbereich, und beträgt beispielsweise zwischen 50 und 80 Grad.

Vorzugsweise weist die Öffnung, welche den Einsatz aufnimmt, zumindest einen Abschnitt mit einer nicht-rotationssymmetrischen Innenkontur auf. Dieser Abschnitt ist so geformt, dass der erste Abschnitt des Einsatzes in der nicht-rotationssymmetrischen Innenkontur der Öffnung verdrehsicher aufgenommen werden kann.

Gemäß einer alternativen Ausgestaltung weist die Öffnung, welche den Einsatz aufnimmt, zumindest einen Abschnitt mit einer nicht-rotationssymmetrischen Innenkontur aufm welcher so geformt ist, dass die nicht-rotationssymmetrische Außenkontur des ersten Abschnitt des Einsatzes beim Einpressvorgang des Einsatzes in die Öffnung geformt wird. Dies wird beispielsweise durch eine Zahnform des nicht-rotationssymmetrischen Innenkontur-Abschnitts der Öffnung realisiert. Dabei können die Zähne einen Verlauf in Richtung der Oberseite der Osteosyntheseplatte aufweisen, sodass ein gleichmäßiges Einformen der nicht-rotationssymmetrischen Außenkontur des Einsatzes erzielt wird.

Vorzugsweise weist die Osteosyntheseplatte mehrere runde Öffnungen auf, wobei in jedem der runden Öffnungen ein Einsatz wie oben beschrieben angeordnet ist. Die Osteosyntheseplatte kann auch nicht-runde Öffnungen wie beispielsweise ein Langloch aufweisen. Das Anordnen von einem Einsatz in jeder runden Öffnung erleichtert die Verwendung der Osteosyntheseplatte, da der Operateur keine verschiedenen Anweisungen zur Nutzung der runden Öffnungen berücksichtigen muss.

Zur Lösung der weiteren Aufgabe wird ein Verfahren zur Herstellung einer eingangs beschriebenen Osteosyntheseplatte mit folgenden Schritten vorgeschlagen:
- Bereitstellen des Grundkörpers und des zumindest einen Einsatzes;
- Einpressen oder Einsetzen des Einsatzes in einer der Öffnungen des Grundkörpers ausgehend von der Oberseite des Grundkörpers; und
- Umformen zumindest eines Bereichs des zweiten Abschnitts des Einsatzes zur Herstellung einer formschlüssigen Verbindung zwischen dem Einsatz und dem Grundkörper, ausgehend von der Unterseite des Grundkörpers.

Ein derartiges Verfahren ermöglicht ein prozess-sicheres und zuverlässiges Verankern des Einsatzes in der Öffnung.

Vorzugsweise wird im Schritt "Einpressen oder Einsetzen des Einsatzes" ein ursprünglich rotationssymmetrischer Bereich des Einsatzes zur nicht-rotationssymmetrischen Außenkontur des Einsatzes geformt. In anderen Worten entsteht die nicht-rotationssymmetrische Außenkontur des ersten Abschnitts des Einsatzes erst während dem Fügevorgang des Einsatzes in die Öffnung. Dies reduziert den Fertigungsaufwand zur Herstellung des Einsatzes.

Gemäß einer alternativen Ausgestaltung umfasst der Schritt "Bereitstellen des zumindest eines Einsatzes" einen Schritt zur Herstellung der nicht-rotationssymmetrischen Außenkontur des ersten Abschnitts des Einsatzes. In anderen Worten entsteht die nicht-rotationssymmetrische Außenkontur des Einsatzes nicht erst beim Fügevorgang des Einsatzes in die Öffnung, sondern bereits in der Fertigung des Einsatzes selbst. Die nicht-rotationssymmetrische Außenkontur des ersten Abschnitts des Einsatzes kann beispielsweise durch spanende Bearbeitung, durch Sintern oder durch Einrollen erfolgen.

Der Grundkörper der Osteosyntheseplatte ist vorzugsweise aus einer Titan- oder Stahllegierung gefertigt. Der oder die Einsätze ist vorzugsweise aus einer Titanlegierung oder aus Reintitan gefertigt, beispielsweise aus Titan Grade 2. Der Grundkörper und/oder der oder die Einsätze können oberflächenbehandelt sein, beispielsweise anodisiert. Der oder die Einsätze können eine unterschiedliche Oberflächenbehandlung aufweisen, beispielsweise zur farbigen Kenntlichmachung unterschiedlicher kompatibler Schrauben.

Ausführungsbeispiele der Erfindung sind anhand der Figuren detailliert beschrieben. Es zeigen:
- Fig. 1-3: je eine isometrische Ansicht einer Osteosyntheseplatte gemäß einer ersten Ausführungsform;
- Fig. 3b: eine isometrische Ansicht der Osteosyntheseplatte gemäß einer ersten Ausführungsform mit Schrauben;
- Fig. 4-6: je eine Detailansicht der Osteosyntheseplatte gemäß der ersten Ausführungsform;
- Fig. 7: eine Draufsicht auf einen Einsatz gemäß der ersten Ausführungsform;
- Fig. 8: eine Schnittansicht des Einsatzes gemäß der ersten Ausführungsform;
- Fig. 9-10: je eine Schnittansicht einer Osteosyntheseplatte mit alternativen Ausgestaltungen des Einsatzes;
- Fig. 11: eine Detail-Schnittansicht des Einsatzes gemäß der ersten Ausführungsform;
- Fig. 12: eine Detailansicht einer Öffnung der Osteosyntheseplatte gemäß der ersten Ausführungsform;
- Fig. 13-18: verschiedene Ansichten eines Fügeprozesses des Einsatzes in einen Grundkörper der Osteosyntheseplatte gemäß der ersten Ausführungsform;
- Fig. 19-21: je eine Detailansicht einer Osteosyntheseplatte gemäß einer zweiten Ausführungsform; und
- Fig. 22-24: je eine Schnittansicht der Osteosyntheseplatte gemäß der zweiten Ausführungsform.

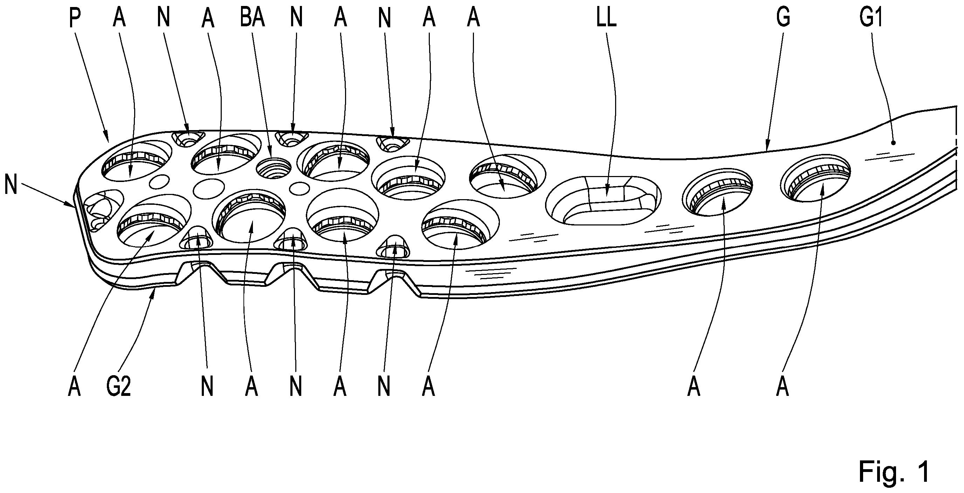

Fig. 1 zeigt eine isometrische Ansicht einer Osteosyntheseplatte P gemäß einer ersten Ausführungsform. Die Osteosyntheseplatte P dient zur Fixierung von in den Figuren nicht dargestellten Knochenfragmenten, und ist beispielhaft für die Anwendung am proximalen Humerus ausgebildet. Die Osteosyntheseplatte P weist einen länglichen Grundkörper G mit einer Oberseite G1 und mit einer der Oberseite G1 gegenüberliegenden Unterseite G2 auf. Die Unterseite G2 ist zur Auflage der Osteosyntheseplatte P auf den Knochenfragmenten vorgesehen. In der Darstellung gemäß Fig. 1 ist links die proximale Seite, und rechts die distale Seite. Die Osteosyntheseplatte P weist mehrere runde Öffnungen A auf, welche zur Aufnahme von je einer Schraube SK eingerichtet sind. Mittels der Schrauben SK kann die Osteosyntheseplatte P auf dem Knochen befestigt werden, um die Knochenfragmente in einer gewünschten Position zueinander zu fixieren. Durch die natürliche Osteosynthese bildet sich im Bereich des/der Spalte zwischen den Knochenfragmenten neues Knochenmaterial. Die Osteosyntheseplatte P weist ferner ein Langloch LL auf, welches ebenfalls zur Aufnahme von einer Schraube SK eingerichtet ist. Die Osteosyntheseplatte P weist mehrere Nahtanker N auf, um Weichgewebe an der Osteosyntheseplatte P befestigen zu können, beispielsweise eine oder mehrere Sehnen. An der Oberseite G1 ist eine Sacklochbohrung BA mit Innengewinde vorgesehen, um einen in Fig. 1 nicht dargestellten Bohrführungsblock daran befestigen zu können. Der Grundkörper G ist aus einem für die Anwendung in der Medizintechnik geeigneten Metall gefertigt, beispielsweise aus einer Titanlegierung oder aus Edelstahl.

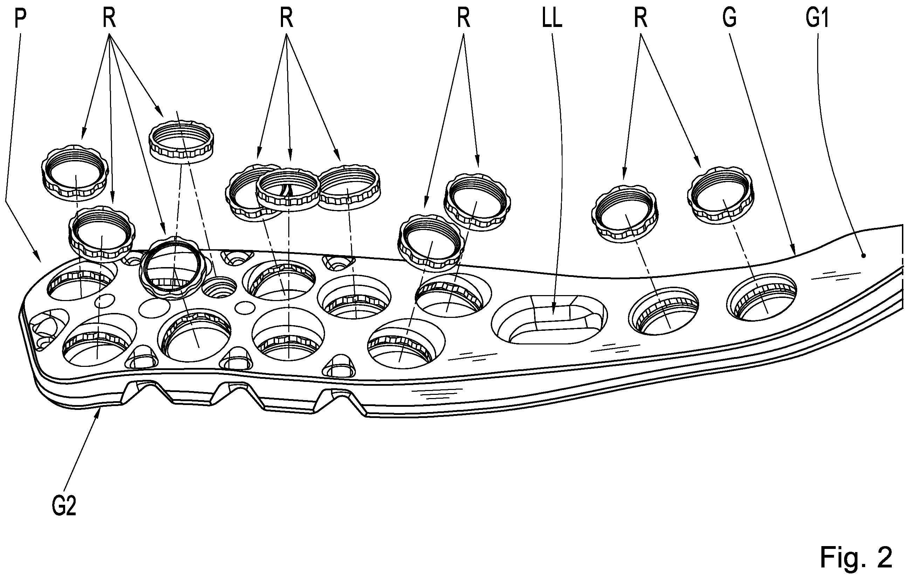

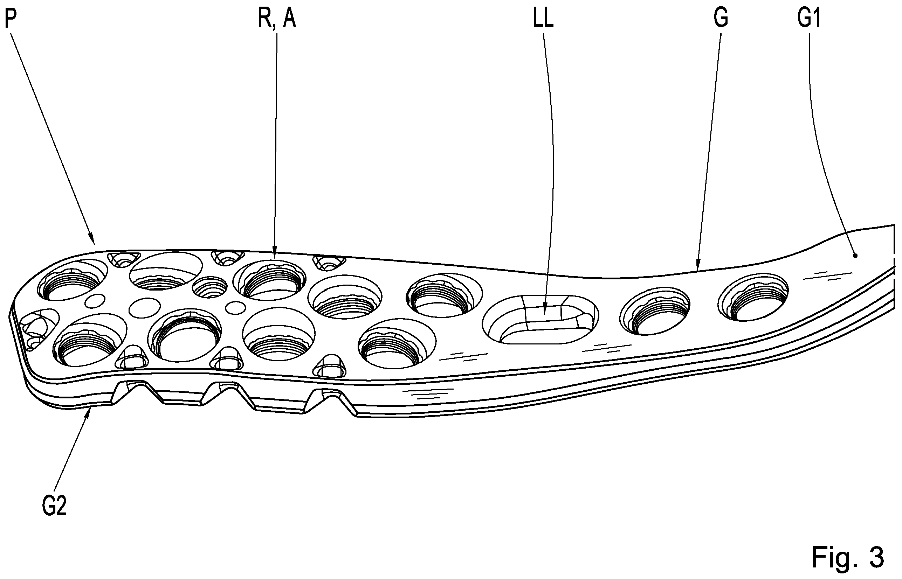

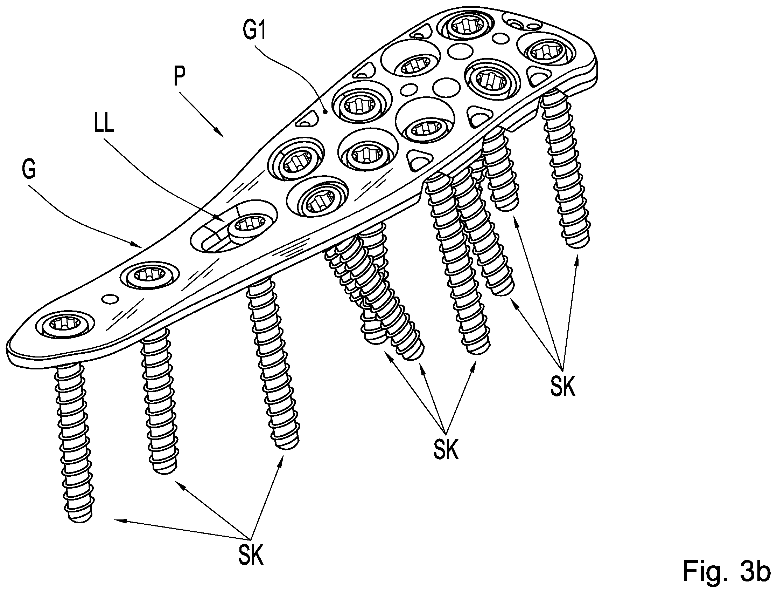

In Fig. 2 ist die Osteosyntheseplatte P mit dazugehörigen Einsätzen R dargestellt. Jeder der Einsätze R ist einer der runden Öffnungen A zugeordnet. Die Einsätze R sind einteilig und ringförmig ausgebildet. Die Einsätze R sind aus einem für die Anwendung in der Medizintechnik geeigneten Metall gefertigt, welches jedoch eine höhere Duktilität aufweist als der Werkstoff des Grundkörpers G, beispielsweise aus einer entsprechend duktilen Titanlegierung oder aus Reintitan. Wie in Fig. 2 dargestellt sind die Achsen der Öffnungen A zur Aufnahme der Einsätze R unterschiedlich ausgerichtet, um eine Vorausrichtung der Schrauben SK bereitzustellen. Die Form der Einsätze R erlaubt eine von dieser Vorausrichtung abweichende Ausrichtung der Schrauben SK. Dem Langloch LL ist kein Einsatz zugeordnet. Das Langloch LL ist derart ausgebildet, dass eine Winkelausrichtung der dem Langloch LL zugeordneten Schraube SK vorgegeben ist. Fig. 3 zeigt die Osteosyntheseplatte P mit in den Öffnungen A angeordneten Einsätzen R.

Fig. 3b zeigt eine weitere Ansicht der Osteosyntheseplatte P, wobei in sämtlichen runden Öffnungen A sowie im Langloch LL je eine Schraube SK angeordnet ist. Die Schrauben SK sind in den Öffnungen A sowie im Langloch LL am Schraubenkopf aufgenommen, welcher ein in Fig. 3b nicht sichtbares Außengewinde aufweist.

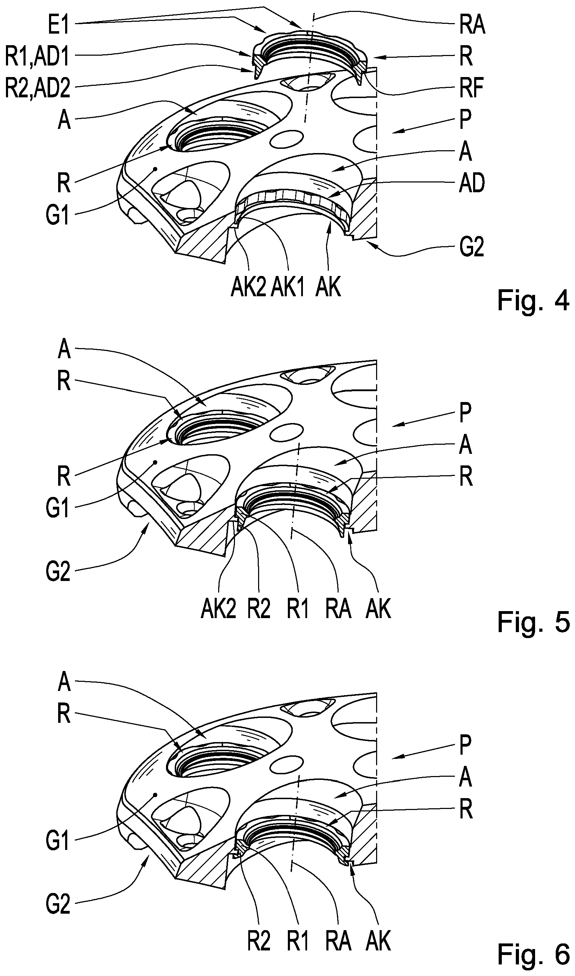

Fig. 4 zeigt eine Detailansicht der Osteosyntheseplatte P in einer Explosionsdarstellung, um die Aufnahme des Einsatzes R in der Öffnung A während dem Montageprozess der Osteosyntheseplatte P zu visualisieren. Zur besseren Verdeutlichung sind Teile des Grundkörpers G sowie des Einsatzes R geschnitten dargestellt. Die Öffnung A weist eine nicht-rotationssymmetrische Innenkontur AD sowie einen radial nach innen gerichteten Vorsprung AK auf. Der Vorsprung AK weist eine zur Oberseite G1 gerichtete erste Fläche AK1 sowie eine zur Unterseite G2 gerichtete zweite Fläche AK2 auf. Der Einsatz R weist einen ersten Abschnitt R1 und einen zweiten Abschnitt R2 auf. Der erste Abschnitt R1 ist der Oberseite G1 zugewandt, und weist eine nicht-rotationssymmetrische Außenkontur AD1 in Form von mehreren am Umfang verteilten radialen Erhebungen E1 auf. Der zweite Abschnitt R2 ist der Unterseite G2 zugewandt, und weist eine rotationssymmetrische Außenkontur AD2 auf. Der Einsatz R weist eine Fläche RF auf, welche orthogonal zur Mittelachse RA des Einsatzes R ausgerichtet ist.

Fig. 5 zeigt eine Detailansicht der Osteosyntheseplatte P, in welcher der Einsatz R in die Öffnung A eingeführt wurde, sodass die Fläche RF auf dem Vorsprung AK aufliegt. Die nicht-rotationssymmetrische Außenkontur AD2 des Einsatzes R ist in die dazu komplementär gebildete nicht-rotationssymmetrische Innenkontur AD der Öffnung A eingeführt. Dadurch wird eine Verdrehsicherung des Einsatzes R relativ zum Grundkörper G erreicht. Der zweite Abschnitt R2 des Einsatzes R ragt in Richtung der Unterseite G2 über die zweite Fläche AK2 des Vorsprungs AK hinaus.

Fig. 6 zeigt eine Detailansicht der Osteosyntheseplatte P, in welcher der Einsatz R in die Öffnung A eingeführt wurde, und wobei der zweite Abschnitt R2 so umgeformt wurde, dass der zweite Abschnitt R2 zumindest teilweise auf der zweiten Fläche AK2 aufliegt, sodass der Vorsprung AK von beiden Stirnseiten eingefasst ist. Dadurch wird eine Axialsicherung des Einsatzes R relativ zum Grundkörper G erreicht.

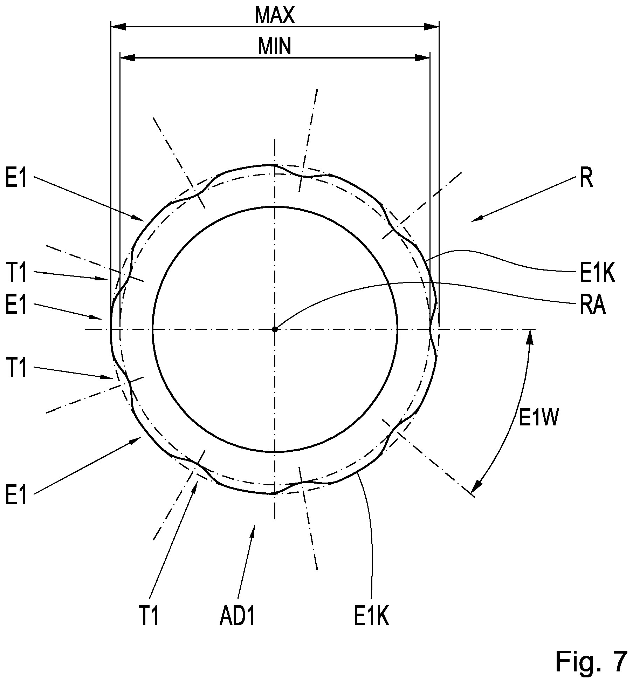

Fig. 7 zeigt eine Draufsicht auf den Einsatz R, sodass der erste Abschnitt R1 des Einsatzes R sichtbar ist. Die nicht-rotationssymmetrische Außenkontur AD1 des ersten Abschnitts R1 wird durch neun gleichmäßig am Umfang verteilte radiale Erhebungen E1 gebildet. Die nicht-rotationssymmetrische Außenkontur AD1 ist dabei wellenförmig ausgebildet, sodass sich die radialen Erhebungen E1 mit Tälern T1 abwechseln. Die Übergänge zwischen den Erhebungen E1 und den Tälern T1 verlaufen in tangentialer Richtung. Die Erhebungen E1 bilden dabei einen größeren Anteil der nicht-rotationssymmetrische Außenkontur AD1 als die Täler T1. Jede der Erhebungen E1 weist einen kreisbogenförmigen Abschnitt E1K auf, dessen Zentrum koaxial zur Mittelachse RA des Einsatzes R ist. Die Erhebungen E1 sind gleichmäßig am Umfang verteilt, sodass jede der Erhebungen E1 um den gleichen Winkel E1W versetzt ist. Das Verhältnis zwischen einem kleinsten Durchmesser MIN der Täler T1 und dem größten Durchmesser MAX der Erhebungen E1 ist größer als 0,9.

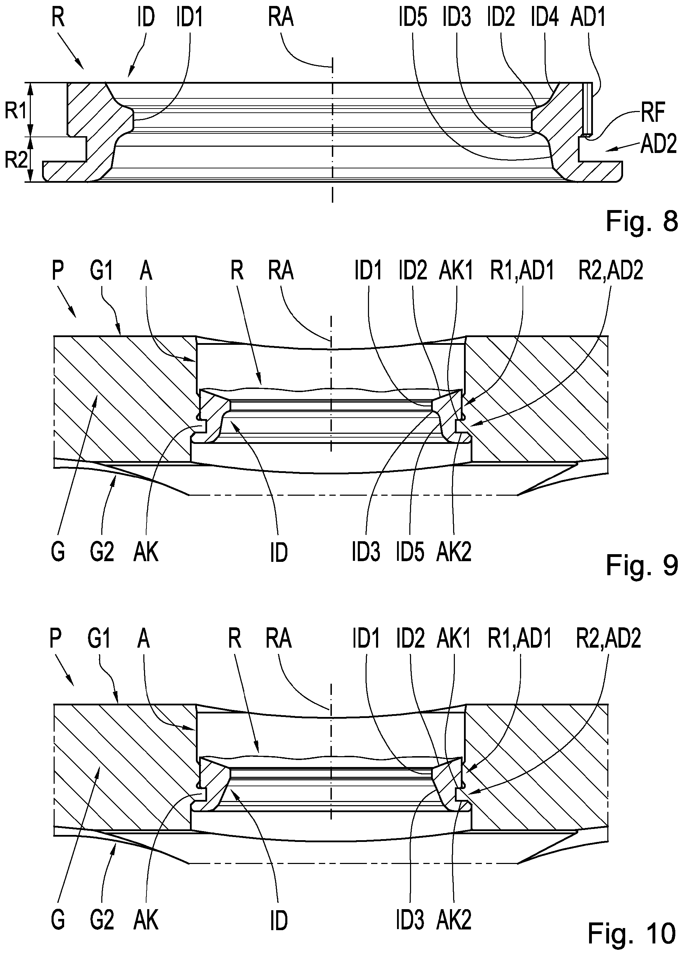

Fig. 8 zeigt eine Schnittansicht des Einsatzes R, wobei der zweite Abschnitt R2 des Einsatzes R wie in Fig. 6 dargestellt bereits umgeformt ist, um eine Axialsicherung des Einsatzes R relativ zum Grundkörper G bereitzustellen. In Fig. 8 ist deutlich zu erkennen, dass der Einsatz R eine rotationssymmetrische Innenkontur ID mit nicht-konstantem Innendurchmesser aufweist. Die Innenkontur ID weist einen ersten Abschnitt ID1 mit konstantem Durchmesser auf, welcher den kleinsten Durchmesser der Innenkontur ID bildet. Bei der Befestigung der Osteosyntheseplatte P mit den Schrauben SK an den Knochenfragmenten wird im ersten Abschnitt ID1 vom Außengewinde des Schraubenkopfs ein Gewinde eingeformt. Ausgehend vom ersten Abschnitt ID1 weist die Innenkontur ID in Richtung der Oberseite G1 einen zweiten Abschnitt ID2 auf. Der zweite Abschnitt ID2 bildet eine linear kegelig geformte Innenkontur. Ausgehend vom ersten Abschnitt ID1 weist die Innenkontur ID in Richtung der Unterseite G2 einen dritten Abschnitt ID3 auf. Der dritte Abschnitt ID3 bildet eine linear kegelig geformte Innenkontur. Die Abschnitte ID2, ID3 bilden einen verhältnismäßig flachen Kegelwinkel, um ein Fließverhalten des Einsatzes R beim Einformen des Gewindes zu verbessern. Ausgehend vom zweiten Abschnitt ID2 weist die Innenkontur ID in Richtung der Oberseite G1 einen vierten Abschnitt ID4 auf. Der vierte Abschnitt ID4 bildet eine linear kegelig geformte Innenkontur. Ausgehend vom dritten Abschnitt ID3 weist die Innenkontur ID in Richtung der Unterseite G2 einen fünften Abschnitt ID5 auf. Der fünfte Abschnitt ID5 bildet eine linear kegelig geformte Innenkontur. Die Innenkontur ID des Einsatzes R erlaubt eine Verankerung der Schraube SK in verschiedenen Winkeln relativ zur Mittelachse RA des Einsatzes R.

Fig. 9 zeigt eine Schnittansicht der Osteosyntheseplatte P mit einer alternativen Ausgestaltung des Einsatzes R. Im Vergleich zur in Fig. 8 dargestellten Ausführung weist der Einsatz R keinen vierten Abschnitt ID4 auf. Stattdessen erstreckt sich der zweite Abschnitt ID2 bis zur jener Stirnseite des Einsatzes R, welcher zur Oberseite G1 der Osteosyntheseplatte P gerichtet ist.

Fig. 10 zeigt eine Schnittansicht der Osteosyntheseplatte P mit einer weiteren alternativen Ausgestaltung des Einsatzes R. Im Vergleich zur in Fig. 9 dargestellten Ausführung weist der Einsatz R keinen fünften Abschnitt ID5 auf. Stattdessen erstreckt sich der dritte Abschnitt ID3 bis zur jener Stirnseite des Einsatzes R, welcher zur Unterseite G2 der Osteosyntheseplatte P gerichtet ist.

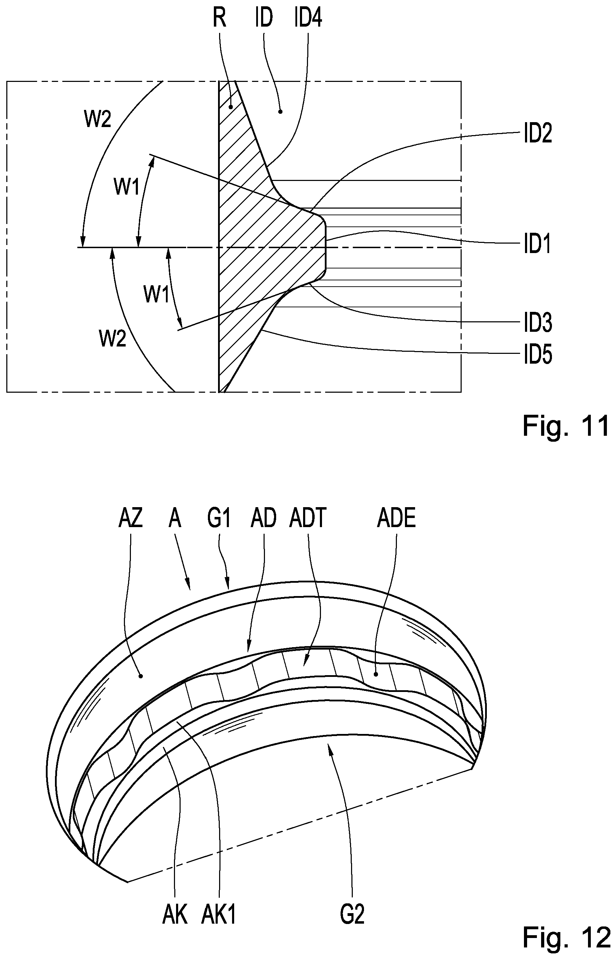

Fig. 11 zeigt eine Detailansicht des Einsatzes R zur Darstellung der Innenkontur ID im Bereich des ersten Abschnitts ID1. Der Einsatz R ist wie in Fig. 8 dargestellt ausgeführt. Aus der Darstellung gemäß Fig. 11 wird deutlich, dass die unmittelbar an den ersten Abschnitt ID1 anschließenden kegeligen Abschnitte ID2, ID3 einen flacheren Kegelwinkelbereich W1 aufweisen als die daran anschließenden kegeligen Abschnitte ID4, ID5, welche einen steileren Kegelwinkelbereich W2 aufweisen.

Fig. 12 zeigt eine Detailansicht der Öffnung A zur Darstellung der nicht-rotationssymmetrischen Innenkontur AD sowie des Vorsprungs AK. Die nicht-rotationssymmetrische Innenkontur AD weist radial nach innen gerichtete Erhebungen ADE auf, welche zwischen zylindrisch geformten Abschnitten ADT hervorstehen. Ausgehend von der nicht-rotationssymmetrischen Innenkontur AD weist die Öffnung A in Richtung der Oberseite G1 einen zylindrischen Abschnitt AZ auf. Die der Oberseite G1 zugewandte Fläche AK1 des Vorsprungs AK schließt unmittelbar an die nicht-rotationssymmetrische Innenkontur AD an.

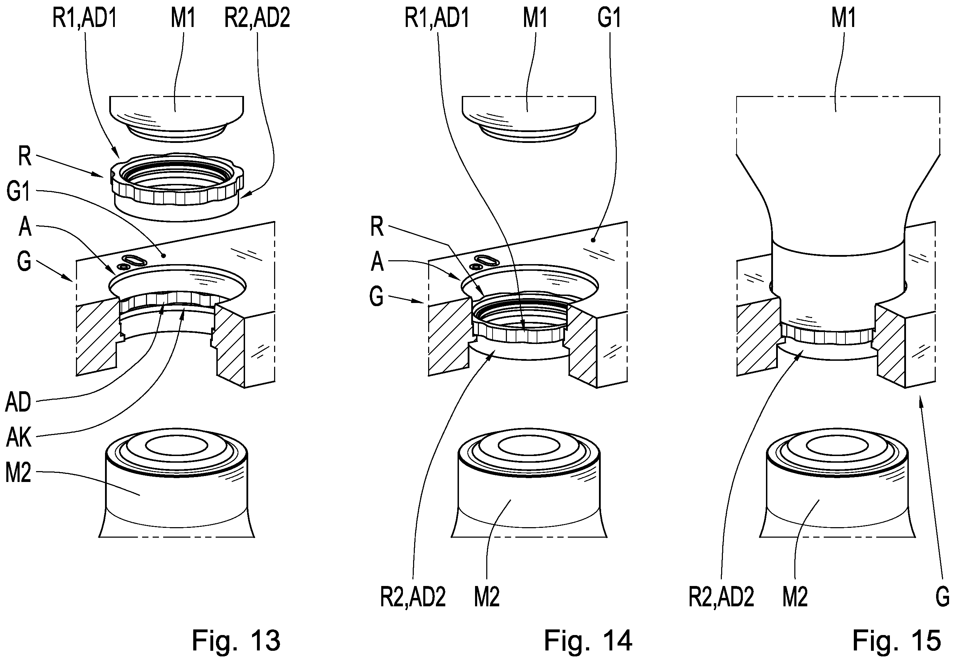

Fig. 13 bis 18 zeigen verschiedene Ansichten eines Fügeprozesses des Einsatzes R in den Grundkörper G der Osteosyntheseplatte P. In der Ansicht gemäß Fig. 13 wird der Grundkörper G und der Einsatz R zwischen zwei Werkzeugen M1, M2 bereitgestellt. In der Ansicht gemäß Fig. 14 ist der Einsatz R in die Öffnung A des Grundkörpers G eingelegt, sodass die nicht-rotationssymmetrische Außenkontur AD1 in der komplementär ausgebildeten nicht-rotationssymmetrischen Innenkontur AD aufgenommen ist. In der Ansicht gemäß Fig. 15 wird der Einsatz R mittels dem Werkzeug M1 in die Öffnung A eingepresst, sodass der Einsatz R am radial nach innen gerichteten Vorsprung AK aufliegt.

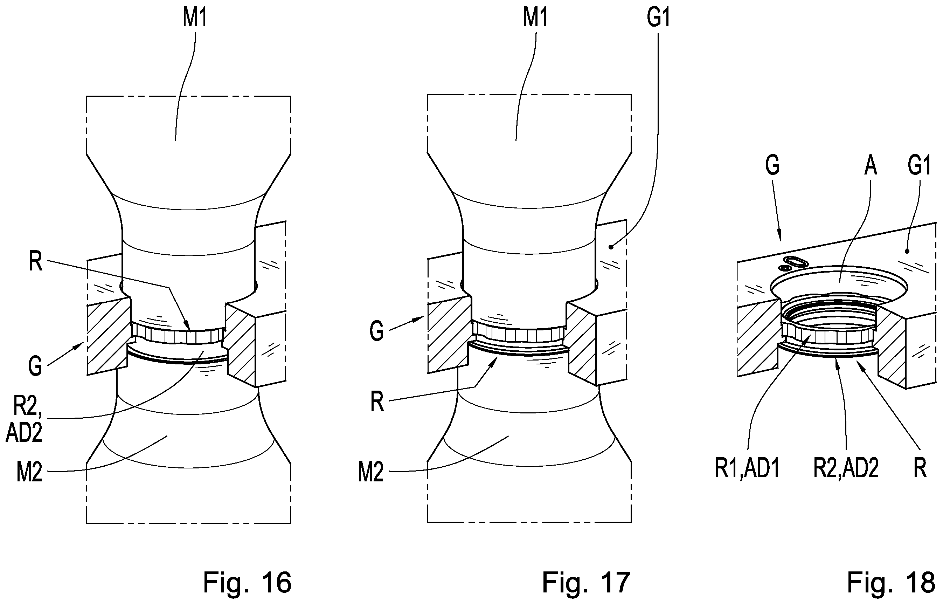

In der Ansicht gemäß Fig. 16 wird das Werkzeug M2 ausgehend von der Unterseite G2 in Richtung des zweiten Abschnitts R2 des Einsatzes R herangeführt, sodass das Werkzeug M2 am Einsatz R aufliegt. In der Ansicht gemäß Fig. 17 wird der zweite Abschnitt R2 des Einsatzes R durch das Werkzeug M2 umgeformt, sodass der radial nach innen gerichtete Vorsprung AK von beiden Stirnseiten vom Einsatz R umschlossen ist. Dabei wird der Einsatz R in Richtung der Oberseite G1 vom Werkzeug M1 gegengehalten. Fig. 18 zeigt den fertig montierten Einsatz R nach Lösen der Werkzeuge M1, M2.

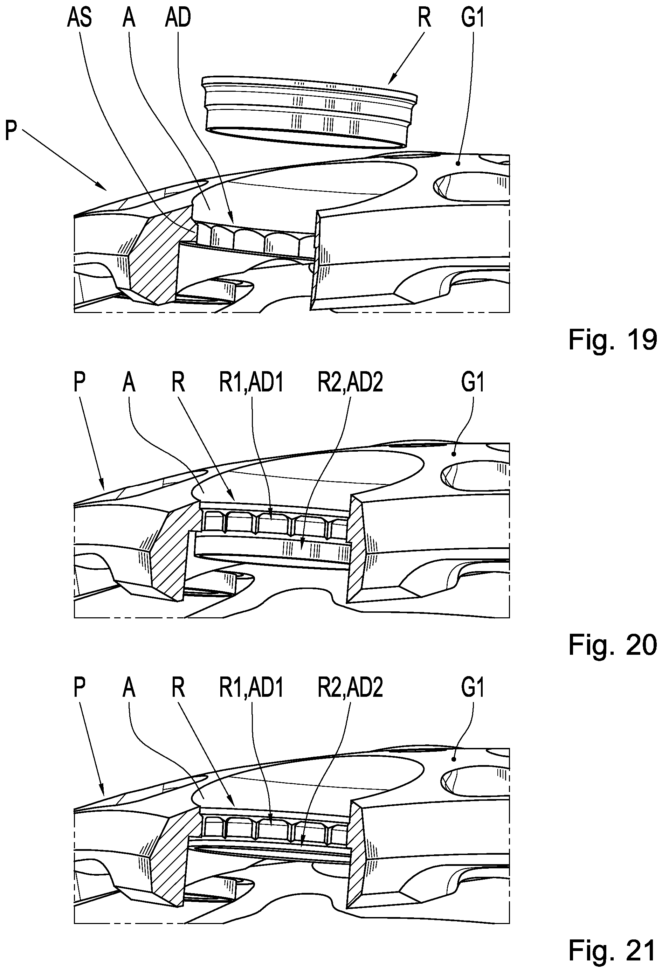

Fig. 19 zeigt eine Detailansicht einer Osteosyntheseplatte P gemäß einer zweiten Ausführungsform. Die nicht-rotationssymmetrische Innenkontur ID der Öffnung A weist dabei eine Stufe AS auf, welche eine Verzahnung bildet. Der Einsatz R weist im in Fig. 19 dargestellten nicht-montierten Zustand noch keine nicht-rotationssymmetrische Außenkontur auf. Stattdessen ist der Einsatz R im nicht montierten Zustand rotationssymmetrisch ausgebildet.

Fig. 20 zeigt eine Detailansicht der Osteosyntheseplatte P gemäß der zweiten Ausführungsform, in der der Einsatz R im Grundkörper G der Osteosyntheseplatte P eingeführt ist. Durch das Einführen des Einsatzes R in die Verzahnung der Stufe AS wird die nicht-rotationssymmetrische Außenkontur AD1 am ersten Abschnitt R1 des Einsatzes R gebildet. Der räumlich darunterliegende zweite Abschnitt R2 weist einen kleineren Durchmesser auf, sodass der zweite Abschnitt R2 durch die Verzahnung der Stufe AS nicht umgeformt wird.

Fig. 21 zeigt eine Detailansicht der Osteosyntheseplatte P gemäß der zweiten Ausführungsform, in der der Einsatz R im Grundkörper G der Osteosyntheseplatte P fertig montiert ist. Der zweite Abschnitt R2 ist nun umgeformt, sodass der zweite Abschnitt R2 eine zur Unterseite G2 gerichtete Fläche der Stufe AS umschließt. Dadurch wird eine Axialsicherung des Einsatzes R relativ zum Grundkörper G erreicht.

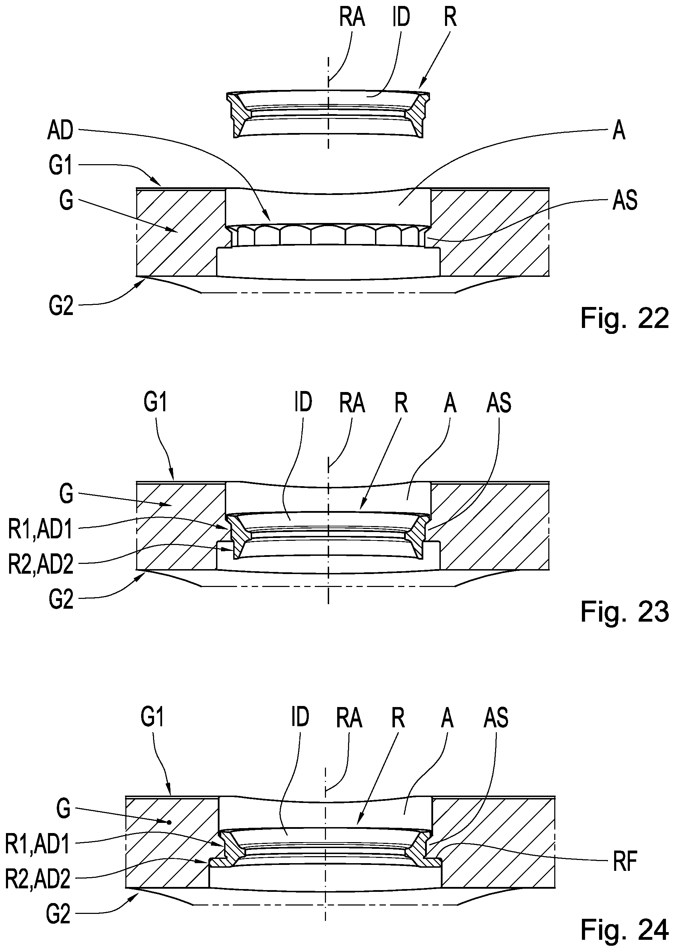

Fig. 22 bis Fig. 24 zeigen verschiedene Schnittansichten eines Abschnitts der Osteosyntheseplatte P gemäß der zweiten Ausführungsform während der Montage des Einsatzes R in den Grundkörper G, jedoch ohne Darstellung der Werkzeuge. In der Ansicht gemäß Fig. 22 ist ein Zustand dargestellt, in der der Einsatz R noch nicht im Grundkörper G montiert ist. In der Darstellung gemäß Fig. 22 ist die Stufe AS mit der daran ausgebildeten Verzahnung deutlich sichtbar. In der Ansicht gemäß Fig. 23 ist ein Zustand dargestellt, in der der Einsatz R in den Grundkörper G eingeführt wurde, sodass die nicht-rotationssymmetrische Außenkontur AD1 am ersten Abschnitt R1 des Einsatzes R hergestellt ist. Der zweite Abschnitt R2 ist in diesem Zustand noch nicht umgeformt. In der Ansicht gemäß Fig. 24 ist ein Zustand dargestellt, in der der zweite Abschnitt R2 des Einsatzes R umgeformt wurde, sodass eine Fläche RF am Übergang zwischen dem ersten und zweiten Abschnitts R1, R2 gebildet wird, welche orthogonal zur Mittelachse RA des Einsatzes R ausgerichtet ist, und auf einer der Unterseite G2 zugewandten Stirnseite der Stufe AS aufliegt.

Die Innenkontur ID des Einsatzes R gemäß der zweiten Ausführungsform entspricht beispielhaft der in Fig. 8 dargestellten Innenkontur ID des Einsatzes R gemäß der ersten Ausführungsform. Dies ist nur beispielhaft anzusehen. Der Innenkontur ID des Einsatzes R gemäß der zweiten Ausführungsform könnte auch wie in Fig. 9 oder Fig. 10 dargestellt ausgebildet sein.

### Bezugszeichenliste

- P: Osteosyntheseplatte
- SK: Schraube
- G: Grundkörper
- G1: Oberseite
- G2: Unterseite
- N: Nahtanker
- BA: Sacklochbohrung
- A: Öffnung
- AK: Vorsprung
- AK1: Erste Fläche
- AK2: Zweite Fläche
- AD: Nicht-rotationssymmetrische Innenkontur
- ADE: Erhebungen
- ADT: Zylindrische Abschnitte
- AZ: Zylindrischer Abschnitt
- AS: Stufe
- LL: Langloch
- R: Einsatz
- RA: Mittelachse
- R1: Erster Abschnitt des Einsatzes
- AD1: Nicht-rotationssymmetrische Außenkontur
- E1: Erhebungen
- E1W: Winkel
- MIN: Kleinster Durchmesser
- MAX: Größer Durchmesser
- E1K: Kreisbogenförmiger Abschnitt
- T1: Täler
- R2: Zweiter Abschnitt des Einsatzes
- AD2: Rotationssymmetrische Außenkontur
- RF: Fläche
- ID: Innenkontur
- ID1: Erster Abschnitt
- ID2: Zweiter Abschnitt
- ID3: Dritter Abschnitt
- ID4, ID5: Weiterer Abschnitt
- W1: Erster Kegelwinkelbereich
- W2: Zweiter Kegelwinkelbereich
- M1, M2: Werkzeug

## Patentansprüche

1. Osteosyntheseplatte (P) zur Fixierung von Knochenfragmenten, wobei die Osteosyntheseplatte (P) einen länglichen Grundkörper (G) mit einer Oberseite (G1) und mit einer der Oberseite (G1) gegenüberliegenden Unterseite (G2) aufweist, wobei die Unterseite (G2) zur Auflage der Osteosyntheseplatte (P) auf den Knochenfragmenten vorgesehen ist, wobei die Osteosyntheseplatte (P) mehrere Öffnungen (A) zur Aufnahme von je einer Schraube (SK) aufweist,
wobei in zumindest einer der Öffnungen (A) ein einteilig und ringförmig ausgebildeter Einsatz (R) angeordnet ist, wobei der Einsatz (R) einen ersten und einen zweiten Abschnitt (R1, R2) aufweist, wobei der erste Abschnitt (R1) der Oberseite (G1) zugewandt ist und in axialer Richtung unmittelbar auf den zweiten Abschnitt (R2) anschließend angeordnet ist,
wobei der erste Abschnitt (R1) eine nicht-rotationssymmetrische Außenkontur (AD1) aufweist und der zweite Abschnitt (R2) eine rotationssymmetrische Außenkontur (AD2) aufweist.

2. Osteosyntheseplatte (P) nach Anspruch 1, **dadurch gekennzeichnet, dass** die nicht-rotationssymmetrische Außenkontur (AD1) durch zumindest sechs, vorzugsweise genau acht oder genau neun, gleichmäßig oder ungleichmäßig am Umfang verteilte radiale Erhebungen (E1) gebildet ist.

3. Osteosyntheseplatte (P) nach Anspruch 2, **dadurch gekennzeichnet, dass** die Erhebungen (E1) einen größeren Anteil der nicht-rotationssymmetrischen Außenkontur (AD1) bilden als sich in Umfangsrichtung zwischen den Erhebungen (E1) erstreckenden Täler (T1), und/oder wobei jede der Erhebungen (E1) einen kreisbogenförmigen Abschnitt (E1K) aufweist, dessen Zentrum koaxial zu einer Mittelachse (RA) des Einsatzes (R) ist.

4. Osteosyntheseplatte (P) nach Anspruch 3, **dadurch gekennzeichnet, dass** ein Verhältnis zwischen dem kleinsten Durchmesser (MIN) der zwischen den Erhebungen (E1) angeordneten Abschnitte und dem größten Durchmesser (MAX) der Erhebungen (E1) größer als 0,8, bevorzugt größer als 0,85, besonders bevorzugt größer als 0,9 ist.

5. Osteosyntheseplatte (P) nach einem der Ansprüche 1 bis 4, **dadurch gekennzeichnet, dass** die nicht-rotationssymmetrische Außenkontur (AD1) wellenförmig ausgebildet ist.

6. Osteosyntheseplatte (P) nach einem der Ansprüche 1 bis 5, **dadurch gekennzeichnet, dass** ein Übergang zwischen dem ersten und zweiten Abschnitt (R1, R2) des Einsatzes (R) durch eine Fläche (RF) gebildet ist, welche orthogonal zur Mittelachse (RA) des Einsatzes (R) ausgerichtet ist, wobei die Fläche (RF) vorzugsweise an einem radial nach innen gerichteten Vorsprung (AK) der Öffnung (A) oder an einer Stufe (AS) der Öffnung (A) anliegt.

7. Osteosyntheseplatte (P) nach einem der Ansprüche 1 bis 6, **dadurch gekennzeichnet, dass** die Öffnung (A) einen radial nach innen gerichteten Vorsprung (AK) aufweist, welcher eine zur Oberseite (G1) der Osteosyntheseplatte (P) gerichtete erste Fläche (AK1) und eine zur Unterseite (G2) der Osteosyntheseplatte (P) gerichtete zweite Fläche (AK1) aufweist, wobei der zweite Abschnitt (R2) des Einsatzes (R) zumindest bereichsweise sowohl auf der ersten Fläche (AK1) als auch auf der zweiten Fläche (AK2) aufliegt.

8. Osteosyntheseplatte (P) nach einem der Ansprüche 1 bis 7, **dadurch gekennzeichnet, dass** der Einsatz (R) eine rotationssymmetrische Innenkontur (ID) mit nicht-konstantem Innendurchmesser aufweist.

9. Osteosyntheseplatte (P) nach Anspruch 8, **dadurch gekennzeichnet, dass** die Innenkontur (ID) des Einsatzes (R) einen ersten Abschnitt (ID1) mit konstantem Durchmesser aufweist, welcher den kleinsten Durchmesser der Innenkontur (ID) bildet.

10. Osteosyntheseplatte (P) nach Anspruch 9, **dadurch gekennzeichnet, dass** die Innenkontur (ID) ausgehend vom ersten Abschnitt (ID1) in Richtung der Oberseite (G1) der Osteosyntheseplatte (P) einen zweiten Abschnitt (ID2), und in Richtung der Unterseite (G2) der Osteosyntheseplatte (P) einen dritten Abschnitt (ID3) aufweist, wobei der zweite und dritte Abschnitt (ID2, ID3) zumindest bereichsweise eine linear kegelig geformte Innenkontur mit einem ersten Kegelwinkelbereich (W1) bilden.

11. Osteosyntheseplatte (P) nach Anspruch 10, **dadurch gekennzeichnet, dass** die Innenkontur (ID) ausgehend vom zweiten Abschnitt (ID2) in Richtung der Oberseite (G1) und/oder vom dritten Abschnitt (ID3) in Richtung der Unterseite (G2) einen weiteren Abschnitt (ID4, ID5) aufweist, welcher zumindest bereichsweise eine linear kegelig geformte Kontur mit einem zweiten Kegelwinkelbereich (W2) bilden, wobei der zweite Kegelwinkelbereich (W2) steiler ist als der erste Kegelwinkelbereich (W1).

12. Osteosyntheseplatte (P) nach einem der vorangehenden Ansprüche, **dadurch gekennzeichnet, dass** die Öffnung (A), welche den Einsatz (R) aufnimmt, zumindest einen Abschnitt mit einer nicht-rotationssymmetrischen Innenkontur (AD) aufweist, welche so geformt ist, dass der erste Abschnitt (R1) des Einsatzes (R) in der nicht-rotationssymmetrischen Innenkontur (AD) der Öffnung (A) verdrehsicher aufnehmbar ist.

13. Osteosyntheseplatte (P) nach einem der Ansprüche 1 bis 11, **dadurch gekennzeichnet, dass** die Öffnung (A), welche den Einsatz (R) aufnimmt, zumindest einen Abschnitt mit einer nicht-rotationssymmetrischen Innenkontur (AD) aufweist, welche so geformt ist, dass der erste Abschnitt (R1) des Einsatzes (R) beim Einpressvorgang des Einsatzes (R) in die Öffnung (A) formbar ist.

14. Osteosyntheseplatte (P) nach einem der vorangehenden Ansprüche, **dadurch gekennzeichnet, dass** die Osteosyntheseplatte (P) mehrere runde Öffnungen (A) aufweist, wobei in jeder der runden Öffnungen (A) einer der Einsätze (R) angeordnet ist.

15. Verfahren zur Herstellung einer Osteosyntheseplatte (P) nach einem der vorangehenden Ansprüche, **gekennzeichnet durch** folgende Schritte:
- Bereitstellen des Grundkörpers (G) und des zumindest einen Einsatzes (R);
- Einpressen oder Einsetzen des Einsatzes (R) in einer der Öffnungen (A) des Grundkörpers (G) ausgehend von der Oberseite (G1) des Grundkörpers (G); und
- Umformen zumindest eines Bereichs des zweiten Abschnitts (R2) des Einsatzes (R) zur Herstellung einer formschlüssigen Verbindung zwischen dem Einsatz (R) und dem Grundkörper (G), ausgehend von der Unterseite (G2) des Grundkörpers (G),
wobei im Schritt "Einpressen oder Einsetzen des Einsatzes (R)" ein ursprünglich rotationssymmetrischer Bereich des Einsatzes (R) zur nicht-rotationssymmetrischen Außenkontur (AD1) des Einsatzes (R) geformt wird, oder wobei der Schritt "Bereitstellen des Einsatzes (G)" einen Schritt zur Herstellung der nicht-rotationssymmetrischen Außenkontur (AD1) des ersten Abschnitts (R1) des Einsatzes (R) umfasst.
